# EUROPEAN PATENT SPECIFICATION

(11) **EP 2 131 818 B1**
(45) Date of publication and mention of the grant of the patent: **09.04.2014**
(21) Application number: 08717221.9
(22) Date of filing: 28.02.2008
(51) Int. Cl.: A61K 9/20, A61K 9/50, A61K 9/00, A61K 31/55

(54) **Novel dosage form**
Neue Dosierform
Nouvelle forme dosifiée

(30) Priority: 01.03.2007 US 892267 P
(43) Date of publication of application: 16.12.2009
(73) Proprietor: Glaxo Group Limited, Brentford, Middlesex TW8 9GS (GB)
(72) Inventor: CLARKE, Allan James, Collegeville, Pennsylvania 19426 (US); CULLINGFORD, David Geoffrey, Harlow Essex CM19 5AW (GB); LI, Yu, Collegeville, Pennsylvania 19426 (US)
(74) Representative: Hillier, Mark
(86) International application number: PCT/EP2008/052430
(87) International publication number: WO 2008/104590

(56) References cited:
- WO-A-2004/056369
- WO-A-2005/042491
- WO-A-2005/123723
- WO-A-2006/018260

## Description

This invention relates to a novel dosage form, to a process for preparing the dosage form and to the use of the dosage form in medicine.

International Patent Application, Publication Number WO2004/056369 discloses certain benzazepine derivatives including 1-{6-[(3-cyclobutyl-2,3,4,5-tetrahydro-1*H*-3-benzazepin-7-yl)oxy]-3-pyridinyl}-2-pyrrolidinone.

WO2004/056369 teaches that benzazepine derivatives may be formulated using standard techniques. However, no dosage form containing 1-{6-[(3-cyclobutyl-2,3,4,5-tetrahydro-1*H*-3-benzazepin-7-yl)oxy]-3-pyridinyl}-2-pyrrolidinone is explicitly disclosed. Further relevant prior art includes WO 2005 123723.

In a first aspect, the present invention provides a dosage form comprising:
a) 1-{6-[(3-cyclobutyl-2,3,4,5-tetrahydro-1*H*-3-benzazepin-7-yl)oxy]-3-pyridinyl}-2-pyrrolidinone or a pharmaceutically acceptable salt thereof;
b) a stabiliser, which reduces degradation of 1-{6-[(3-cyclobutyl-2,3,4,5-tetrahydro-1*H*-3-benzazepin-7-yl)oxy]-3-pyridinyl}-2-pyrrolidinone in the dosage form when compared to a dosage form lacking said stabiliser; and
c) a pharmaceutically acceptable excipient.

The dosage form may be adapted for administration to the patient by any desired route of administration. For example, dosage forms include those adapted for (1) oral administration such as tablets, capsules, caplets, pills, lozenges, powders, syrups, elixirs, suspensions, solutions, emulsions, sachets and cachets; (2) parenteral administration such as sterile solutions, suspensions, implants and powders for reconstitution; (3) transdermal administration such as transdermal patches; (4) rectal and vaginal administration such as suppositories, pessaries and foams; (5) inhalation and intranasal administration such as dry powders, aerosols, suspensions, and solutions (sprays and drops); (6) topical administration such as creams, ointments, lotions, solutions, pastes, drops, sprays, foams and gels; (7) ocular such as drops, ointment, sprays, suspensions and inserts; and (8) buccal and sublingual administration such as lozenges, patches, sprays, drops, chewing gums and tablets.

In the context of this invention, the term "pharmaceutically acceptable excipient" refers to any pharmaceutically acceptable material present in the dosage form other than 1-{6-[(3-cyclobutyl-2,3,4,5-tetrahydro-1*H*-3-benzazepin-7-yl)oxy]-3-pyridinyl}-2-pyrrolidinone or a pharmaceutically acceptable salt thereof and the stabiliser. Suitable pharmaceutically acceptable excipients will vary depending upon the particular dosage form chosen and include diluents, binders, disintegrants and superdisintegrants, lubricants, glidants, granulating agents, coating agents, wetting agents, solvents, co-solvents, suspending agents, emulsifiers, sweeteners, flavouring agents, flavour-masking agents, colouring agents, anticaking agents, humectants, chelating agents, plasticizers, viscosity increasing agents, rate modifying agents, preservatives, surfactants. The skilled person will appreciate that certain pharmaceutically acceptable excipients may serve more than one function and may serve alternative functions depending on how much of the excipient is present in the formulation and what other ingredients are present in the formulation. Guidance on the selection of suitable pharmaceutically acceptable excipients is available from Remington's Pharmaceutical Sciences (Mack Publishing Company).

The dosage forms of the invention may be prepared using techniques and methods known to those skilled in the art. Some of the methods commonly used in the art are described in Remington's Pharmaceutical Sciences (Mack Publishing Company).

In one embodiment, the invention is directed to a solid oral dosage form such as a tablet or capsule comprising 1-{6-[(3-cyclobutyl-2,3,4,5-tetrahydro-1*H*-3-benzazepin-7-yl)oxy]-3-pyridinyl}-2-pyrrolidinone or a pharmaceutically acceptable salt thereof, a stabiliser and a diluent. Suitable diluents include saccharides (e.g. lactose, sucrose, dextrose), sugar alcohols (e.g. mannitol, sorbitol), starch (e.g. corn starch, potato starch and pregelatinized starch), cellulose and its derivatives (e.g. microcrystalline cellulose), calcium sulphate and dibasic calcium phosphate. The dosage form may further comprise other conventional excipients such as binders, disintegrants, lubricants and glidants. Suitable binders include starch (e.g. corn starch, potato starch and pregelatinized starch), gelatin, acacia, sodium alginate, alginic acid, tragacanth, guar gum, polyvinylpyrrolidone, and cellulose and its derivatives (e.g. ethylcellulose, methylcellulose, carboxymethylcellulose, hydroxypropylcellulose and hydroxypropylmethylcellulose). Suitable disintegrants include starches, crosslinked polyvinylpyrrolidone, sodium starch glucolate, croscarmellose, alginic acid and sodium carboxymethyl cellulose. Suitable lubricants include stearic acid, magnesium stearate and calcium stearate. Suitable glidants include talc or colloidal silicon dioxide. The oral solid dosage form may further comprise an outer coating which may have cosmetic or functional properties.

In a more particular aspect, the present invention provides a dosage form for oral administration comprising a carrier tablet, which carrier tablet is at least partially covered by a film comprising:
a) 1-{6-[(3-cyclobutyl-2,3,4,5-tetrahydro-1*H*-3-benzazepin-7-yl)oxy]-3-pyridinyl}-2-pyrrolidinone or a pharmaceutically acceptable salt thereof, and
b) a stabiliser that reduces degradation of 1-{6-[(3-cyclobutyl-2,3,4,5-tetrahydro-1*H*-3-benzazepin-7-yl)oxy]-3-pyridinyl}-2-pyrrolidinone in the dosage form, when compared to a dosage form lacking said stabiliser.

In the context of this application, the term "carrier tablet" refers to a tablet that is substantially free of 1-{6-(3-cyclobutyl-2,3,4,5-tetrahydro-1*H*-3-benzazepin-7-yl)oxy]-3-pyridinyl}-2-pyrrolidinone or pharmaceutically acceptable salts thereof. Typically, the tablet does not contain any therapeutic agent, although embodiments in which the carrier tablet contains one or more therapeutic agents are encompassed by the invention.

The composition of the carrier tablet is not important provided that it is pharmaceutically acceptable. The carrier tablet must, however, be of an appropriate size and shape to function as a tablet for oral administration. Any type of tablet may be used, for example, those described in Remington, The Science and Practice of Pharmacy, 21st Edition, 2005 (Ed. D.B. Troy). In one embodiment, the carrier tablet is formed by conventional compression technology and comprises up to 100% w/w diluent, or a mixture of diluents. Conventional diluents include saccharides (e.g. lactose, sucrose, dextrose), sugar alcohols (e.g. mannitol, sorbitol), starch (e.g. corn starch potato starch and pregelatinized starch), cellulose and its derivatives (e.g. microcrystalline cellulose), calcium sulphate and dibasic calcium phosphate. The tablet may further comprise up to 100% w/w binder, or a mixture of binders. Suitable binders include starch (e.g. corn starch, potato starch and pre-gelatinised starch), gelatin, acacia, sodium alginate, alginic acid, tragacanth, guar gum, polyvinylpyrrolidone, and cellulose and its derivatives (e.g. ethylcellulose, methylcellulose, carboxymethylcellulose, hydroxypropylcellulose and hydroxypropylmethylcellulose). Carrier tablets may also contain other conventional excipients such as lubricants (e.g. stearic acid, magnesium stearate and calcium stearate) and glidants (e.g. talc or colloidal silicon dioxide). In one embodiment, lubricants and glidants are each present in an amount up to 10% w/w, more particularly up to 5% w/w. In another embodiment, substrates formed by injection moulding such as moulded tablets or capsule shells may be used as carrier tablets. Suitable thermoplastic materials for injection moulding include hydroxypropylcellulose, ethylcellulose, methacrylates and polyvinyl acetate.

In one embodiment, the carrier tablet is a tablet comprising microcrystalline cellulose (e.g. Avicel PH-102), pregelatinized starch (e.g. Starch 1500) and magnesium stearate. In a more particular embodiment, the carrier tablet has the following composition:

| Excipient | % w/w |
|---|---|
| Microcrystalline Cellulose (e.g. Avicel PH-102) | 90 |
| Pregelatinized starch (e.g. Starch 1500) | 9 |
| Magnesium Stearate | 1 |

In an alternative embodiment, the carrier substrate may be formulated such that it disintegrates in the mouth when administered orally, a so called "orally disintegrating tablet" or "ODT" substrate. Alternatively, the carrier substrate may be formulated so as to disintegrate in water, a so called "fast-dissolve tablet" or "FDT" substrate.

The carrier tablet provides a substrate or support for the film. In one embodiment, the carrier tablets are coated to substantially prevent absorption of 1-{6-[(3-cyclobutyl-2,3,4,5-tetrahydro-1*H*-3-benzazepin-7-yl)oxy]-3-pyridinyl}-2-pyrrolidinone or a pharmaceutically acceptable salt thereof by the carrier tablets. However,-embodiments in which there is absorption of the 1-{6-[(3-cyclobutyl-2,3,4,5-tetrahydro-1*H*-3-benzazepin-7-yl)oxy]-3-pyridinyl}-2-pyrrolidinone or a pharmaceutically acceptable salt thereof by the carrier tablets are encompassed by the invention.

Suitable coatings for carrier tablets include aqueous film coats such as those commercially available from Colorcon, for example, the Opadry® coatings ("OPADRY WHITE 00F18484" or "OPADRY WHITE YS-1-7003"). Other suitable coatings include Surelease® (ethylcellulose). The dosage form may alternatively be coated with a film of gastroresistant and enterosoluble polymeric material. Suitable polymeric materials include cellulose acetophthalate, cellulose acetopropionate, cellulose trimellitate and acrylic and methacrylic copolymers. Colourings can be added.

In one embodiment, the carrier tablet is coated with a film coat to a 2-6% weight gain.

It will be appreciated that, in embodiments where the carrier tablet is coated to substantially prevent absorption of 1-{6-[(3-cyclobutyl-2,3,4,5-tetrahydro-1*H*-3-benzazepin-7-yl)oxy]-3-pyridinyl}-2-pyrrolidinone or a pharmaceutically acceptable salt thereof, the film coat selected must not be soluble in the solvent used during the manufacturing process of the dosage form. For example, where an aqueous solvent system is used, an aqueous film coat (like an Opadry®) will immediately disintegrate and a coating not soluble in water (e.g. Surelease® or Eudragit®) is appropriate.

In one embodiment, the film containing 1-{6-[(3-cyclobutyl-2,3,4,5-tetrahydro-1*H-*3-benzazepin-7-yl)oxy]-3-pyridinyl}-2-pyrrolidinone or a pharmaceutically acceptable salt thereof only partially coats the carrier tablet. In a more particular embodiment, the carrier tablets are shaped to contain one or more recesses or depressions. In such embodiments, the film containing 1-{6-[(3-cyclobutyl-2,3,4,5-tetrahydro-1*H*-3-benzazepin-7-yl)oxy]-3-pyidinyl}-2-pyrrolidinone or a pharmaceutically acceptable salt thereof may be substantially present within the recess of the carrier tablet.

The dosage form and/or film that at least partially covers the carrier tablet comprises 1-{6-[(3-cyclobutyl-2,3,4,5-tetrahydro-1*H*-3-benzazepin-7-yl)oxy]-3-pyridinyl}-2-pyrrolidinone (the "free base") or a pharmaceutically acceptable salt thereof. In the context of this invention, reference to the free base or pharmaceutically acceptable salt encompasses solvates and hydrates of the free base or pharmaceutically acceptable salt.

1-{6-[(3-Cyclobutyl-2,3,4,5-tetrahydro-1*H*-3-benzazepin-7-yl)oxy]-3-pyridinyl}-2-pyrrolidinone may be prepared according to known procedures, such as those disclosed in WO2004/056369. The disclosure of WO2004/056369 is incorporated herein by reference.

Pharmaceutically acceptable acid addition salts of 1-{6-[(3-cyclobutyl-2,3,4,5-tetrahydro-1*H*-3-benzazepin-7-yl)oxy]-3-pyridinyl}-2-pyrrolidinone include hydrobromide, hydrochloride, sulfate, nitrate, phosphate, succinate, maleate, formate, acetate, propionate, fumarate, citrate, tartrate, lactate, benzoate, salicylate, glutamate, aspartate, p-toluenesulfonate, benzenesulfonate, methanesulfonate, ethanesulfonate, naphthalenesulfonate (e.g. 2-naphthalenesulfonate) or hexanoate salts. Such salts can be formed by reaction with the appropriate acid, optionally in a suitable solvent such as an organic solvent, to give the salt which can be isolated for example by crystallisation and filtration.

The dosage form and/or the film may contain the free base, a pharmaceutically acceptable salt (stoichiometric or non-stoichiometric), or any mixture of these. In one embodiment, the dosage form contains the free base. In one embodiment, the film contains the free base.

In one embodiment of the invention, the dosage form and/or the film contains between 1 µg and 1 mg 1-{6-[(3-cyclobutyl-2,3,4,5-tetrahydro-1*H*-3-benzazepin-7-yl)oxy]-3-pyridinyl}-2-pyrrolidinone when measured as the amount of base present (that is, excluding any amount of acid added to form salts). In a more particular embodiment, the dosage form and/or the film contains between 1 µg and 200 µg, more particularly between 1 µg and 100 µg and even more particularly between 2 µg and 100 µg 1-{6-[(3-cyclobutyl-2,3,4,5-tetrahydro-1*H*-3-benzazepin-7-yl)oxy]-3-pyridinyl}-2-pyrrolidinone, when measured as the amount of free base present.

The dosage form and/or the film additionally contains a pharmaceutically acceptable stabiliser that reduces degradation of 1-{6-[(3-cyctobutyl-2,3,4,5-tetrahydro-1*H*-3-benzazepin-7-yl)oxy]-3-pyridinyl}-2-pyrrolidinone (or a pharmaceutically acceptable salt thereof) in the dosage form containing the stabiliser when compared to a dosage form lacking the stabiliser. The degradation of 1-{6-[(3-cyclobutyl-2,3,4,5-tetrahydro-1*H*-3-benzazepin-7-yl)oxy]-3-pyridinyl}-2-pyrrolidinone (or a pharmaceutically acceptable salt thereof) in the dosage form can be analysed by measuring the total impurity/degradation product content of the dosage form using gradient HPLC, using the method described below. The skilled reader will appreciate that, with the exception of the presence or absence of stabiliser, the dosage forms should otherwise be comparable, and should have been stored under similar conditions for similar time periods.

In one embodiment, the mean total impurity/degradation product content calculated from at least 3 samples of the dosage form containing stabiliser stored for 1 month at 40°C, 75% relative humidity is at least 50% lower than the mean total impurity/degradation product content calculated from at least 3 samples of a comparable dosage form lacking said stabiliser that was stored under comparable conditions.

In another embodiment, the mean total impurity/degradation product content of at least 3 samples of the dosage form containing stabiliser when stored at 30°C, 65% relative humidity for a period of 3 months should not exceed 10%, more particularly 5%.

Certain pharmaceutically acceptable antioxidants may act as stabilisers in the context of the present invention. Pharmaceutically acceptable antioxidants include those described in The Handbook of Pharmaceutical Excipients, Third Edition, 2000 (Ed. A.H. Kibbe). In one embodiment, the stabiliser is selected from the group consisting of citric acid, malic acid, ascorbic acid and its salts, sodium bicarbonate, butylated hydroxyanisole and butylated hydroxytoluene. Combinations of stabilisers may also be used in the present invention.

In one embodiment, the stabiliser is citric acid and, optionally butylated hydroxyanisole. In one embodiment, the film contains citric acid and, optionally butylated hydroxyanisole.

The stabiliser or stabilisers must be present in the dosage form and/or the film in a sufficient amount to reduce degradation of 1-{6-[(3-cyclobutyl-2,3,4,5-tetrahydro-1*H*-3-benzazepin-7-yl)oxy]-3-pyridinyl}-2-pyrrolidinone or a pharmaceutically acceptable salt thereof. In certain embodiments comprising citric acid, the molar ratio of free base to citric acid is in the range 1.5 : 1 to 1 : 500.

The film may additionally contain a film former, such as hydroxypropylcellulose, hydroxypropylmethyl cellulose, hydroxyethylcellulose, carboxymethyl cellulose, polyvinyl alcohol, polyvinyl pyrrolidone, carrageenan (kappa iota or lambda), gelatin, polyethylene glycol, polyethylene oxide, pullulan or acrylic polymer (e.g. EUDRAGIT grades RL, RS, E, L, S, FS30D) or any combinations thereof. In one embodiment, the film former is hydroxypropylcellulose. It will be apparent to the one skilled in the art, that any film former included in the dosage form should be soluble in the solvent used during its production.

The film may additionally contain other excipients. For example, it has been found that certain solvent systems, such as aqueous systems, require addition of surfactants (e.g. polysorbates (20, 40, 80), Triton 100, sodium lauryl sulphate or tyloxopol) and/or antifoaming agents (polydimethylsiloxane or dimethicone). Therefore, in a further embodiment, the film additionally contains one or more surfactants and/or one or more antifoaming agents.

The dosage form may be further coated. Suitable coatings include those listed above as suitable for coating of the carrier tablet. In one embodiment, the dosage form is coated to 2-6% weight gain.

The dosage form may optionally be packaged in a low oxygen environment. This may be achieved by inclusion of an oxygen scavenger in the packaging of the dosage form. Suitable oxygen scavengers include PharmaKeep® KH and KD (commercially available from Sud Chemie) and StabilOx™ speciality oxygen scavenger (commercially available from Multisorb Technologies). Alternatively, the dosage forms can be packaged in bottles that are impermeable to oxygen.

Aluminium-aluminium blisters may also be used to package the dosage forms in a low oxygen environment.

In another aspect, the invention provides a method for preparing the dosage form of the invention. The method comprises dispensing a solution or suspension of 1-{6-[(3-cyclobutyl-2,3,4,5-tetrahydro-1*H*-3-benzazepin-7-yl)oxy]-3-pyridinyl}-2-pyrrolidinone or a pharmaceutically acceptable salt thereof and stabiliser onto a carrier tablet. Any solvent may be used provided that the stabiliser and any other excipients present in the film are soluble in the solvent. The solvent is typically volatile, and must be pharmaceutically acceptable in the (residual) quantities in which it appears in the finished dosage form.

Suitable solvents include water, organic solvents, propellants, liquefied gases and volatile silicones. In one embodiment, the solution or suspension of 1-{6-[(3-cyclobutyl-2,3,4,5-tetrahydro-1*H*-3-benzazepin-7-yl)oxy]-3-pyridinyl}-2-pyrrolidinone or a pharmaceutically acceptable salt thereof and stabiliser is prepared using an organic solvent, such as methanol, ethanol, acetone, acetic acid or methylene chloride. Mixtures of solvents (e.g. water ethanol) may also be used. In one embodiment, the solvent is methanol.

In a further aspect, the invention provides a solution or suspension of 1-{6-[(3-cyclobutyl-2,3,4,5-tetrahydro-1*H*-3-benzazepin-7-yl)oxy]-3-pyridinyl}-2-pyrrolidinone or a pharmaceutically acceptable salt thereof and stabiliser in a solvent system. In one embodiment, the solution or suspension further comprises one or more film formers and/or surfactants and/or antifoaming agents. In another embodiment, the solvent is an organic solvent, such as methanol, ethanol, acetone, acetic acid or methylene chloride, more particularly methanol.

In certain embodiments in which the stabiliser is citric acid, it is present in the solution or suspension in an amount between 2-3% w/v, particularly 3% w/v. In certain embodiments in which the stabiliser is butylated hydroxyanisole, it is present in the solution or suspension in an amount between 0.01-0.1% w/v, particularly 0.02% w/v.

In certain embodiments in which the film former is hydroxypropylcellulose, it is present in the solution or suspension in an amount between 4-6% w/v, particularly 4% w/v.

The carrier tablet and dispensed solution/suspension may be heated (e.g. in a forced air oven) to evaporate excessive liquid and result in the formation of a film upon at least a part of the surface of the carrier tablet. The dosage form may then optionally be film coated according to methods known in the art.

The carrier tablet used in the method for preparing the dosage form may have a recess or depression that provides a basin for the solution or suspension of 1-{6-[(3-cyclobutyl-2,3,4,5-tetrahydro-1*H*-3-benzazepin-7-yl)oxy]-3-pyridinyl}-2-pyrrolidinone or a pharmaceutically acceptable salt thereof and stabiliser to land after being dispensed. Typically, biconcave tablets having recesses on two faces of the tablet are employed. The two recesses can be used to receive the solution or suspension of 1-{6-[(3-cyclobutyl-2,3,4,5-tetrahydro-1*H*-3-benzazepin-7-yl)oxy]-3-pyridinyl}-2-pyrrolidinone or a pharmaceutically acceptable salt thereof and stabiliser. Alternatively, one of the recesses can be used to receive a solution or suspension of 1-{6-[(3-cyclobutyl-2,3,4,5-tetrahydro-1*H*-3-benzazepin-7-yl)oxy]-3-pyridinyl}-2-pyrrolidinone or a pharmaceutically acceptable salt thereof and stabiliser, and the remaining recess can be used to receive a solution or suspension of another therapeutic agent to produce a dosage form containing two different therapeutic agents. In a further embodiment, solutions of different therapeutic agents may be layered one on top of the other.

The dosage form of the present invention may be produced using the apparatus described in WO2005/12356 which publication is herein incorporated in its entirety. More particularly, the dosage form of the present invention may be produced by an apparatus containing a dispensing module for accurately dispensing a predetermined amount of the solution or suspension of 1-{6-[(3-cyclobutyl-2,3,4,5-tetrahydro-1*H*-3-benzazepin-7-yl)oxy]-3-pyridinyl}-2-pyrrolidinone or a pharmaceutically acceptable salt thereof and stabiliser onto the carrier tablets. The apparatus may also have a holding member for holding the carrier tablets, which may move continually along the apparatus as the dispensing module dispenses the solution/suspension onto each of the carrier tablets.

The apparatus may also have a drying system that dries or evaporates solvent from the solution/suspension deposited on each of the carrier tablets. The holding member may move continually along the apparatus as the drying system dries the dosage on each of the carrier tablets. The drying system may dry the dosage by use of heated air, infrared or microwave heating.

The apparatus may also have a coating system that applies a coating over the dosage form. The coating system may have a pad printing device or a sprayer that applies the coating to each of the carrier substrates. The holding member may move continually along the apparatus as the coating system applies the coating to each of the carrier tablets. The apparatus may also have a coating dryer that dries the coating on each of the carrier tablets.

It will be apparent that the apparatus described above could re-process carrier tablets any number of times in order to add solutions or suspensions of different therapeutic agents. Alternatively, the apparatus could have additional dispensing systems in series to add each of the solutions/suspensions to the carrier tablets.

1-{6-[(3-Cyclobutyl-2,3,4,5-tetrahydro-1*H*-3-benzazepin-7-yl)oxy]-3-pyridinyl}-2-pyrrolidinone and its pharmaceutically acceptable salts thereof are H3 antagonists having useful therapeutic properties. More particularly, 1-{6-[(3-Cyclobutyl-2,3,4,5-tetrahydro-1*H*-3-benzazepin-7-yl)oxy]-3-pyridinyl}-2-pyrrolidinone and its pharmaceutically acceptable salts are believed to be of potential use in the treatment of neurological diseases including Alzheimer's disease, dementia (including Lewy body dementia and vascular dementia), age-related memory dysfunction, mild cognitive impairment, cognitive deficit, epilepsy, migraine, Parkinson's disease, multiple sclerosis (including fatigue), stroke, pain of neuropathic origin (including neuralgias, neuritis and back pain), inflammatory pain (including osteoarthritis, rheumatoid arthritis, acute inflammatory pain and back pain) and sleep disorders (including hypersomnolence, excessive daytime sleepiness, narcolepsy, sleep deficits associated with Parkinson's disease and fatigue, especially in multiple sclerosis); psychiatric disorders including psychotic disorders (such as schizophrenia (particularly cognitive deficit of schizophrenia) and bipolar disorder), attention deficit hypereactivity disorder, depression (including major depressive disorder), anxiety and addiction; and other diseases including obesity and gastro-intestinal disorders.

Accordingly, in a further aspect, the present invention provides the dosage form for use in therapy. More particularly, the invention provides the dosage form for use in the treatment or prophylaxis of the above disorders, in particular neurological and psychiatric disorders.

The invention further provides a method of treatment or prophylaxis of the above disorders, in mammals including humans, which comprises administering to the sufferer the dosage form of the present invention.

In another aspect, the invention provides the use of 1-{6-[(3-cyclobutyl-2,3,4,5-tetrahydro-1*H*-3-benzazepin-7-yl)oxy]-3-pyridinyl}-2-pyrrolidinone or a pharmaceutically acceptable salt thereof in the manufacture of the dosage form of the invention for use in the treatment of the above disorders.

1-{6-[(3-Cyclobutyl-2,3,4,5-tetrahydro-1*H*-3-benzazepin-7-yl)oxy]-3-pyridinyl}-2-pyrrolidinone or its pharmaceutically acceptable salts may be used in combination with other therapeutic agents. When 1-{6-[(3-Cyclobutyl-2,3,4,5-tetrahydro-1*H*-3-benzazepin-7-yl)oxy]-3-pyridinyl}-2-pyrrolidinone or a pharmaceutically acceptable salt thereof is intended for use in the treatment of Alzheimer's disease, it may be used in combination with medicaments claimed to be useful as either disease modifying or symptomatic treatments of Alzheimer's disease. Suitable examples of such other therapeutic agents may be symptomatic agents, for example those known to modify cholinergic transmission such as M1 muscarinic receptor agonists or allosteric modulators, M2 muscarinic antagonists, acetylcholinesterase inhibitors (such as tetrahydroaminoacridine, donepezil hydrochloride and rivastigmine), nicotinic receptor agonists or allosteric modulators (such as α7 agonists or allosteric modulators or α4β2 agonists or allosteric modulators), PPAR agonists (such as PPARγ agonists), 5-HT₄ receptor partial agonists, 5-HT₆ receptor antagonists or 5HT1A receptor antagonists and NMDA receptor antagonists or modulators, or disease modifying agents such as β or γ-secretase inhibitors.

When 1-{6-[(3-cyclobutyl-2,3,4,5-tetrahydro-1*H*-3-benzazepin-7-yl)oxy]-3-pyridinyl}-2-pyrrolidinone or a pharmaceutically acceptable salt thereof is intended for use in the treatment of narcolepsy, it may be used in combination with medicaments claimed to be useful as treatments for narcolepsy. Suitable examples of such other therapeutic agents include modafinil, armodafinil and monoamine uptake blockers.

When 1-{6-[(3-cyclobutyl-2,3,4,5-tetrahydro-1*H*-3-benzazepin-7-yl)oxy]-3-pyridinyl}-2-pyrrolidinone or a pharmaceutically acceptable salt thereof is intended for use in the treatment of schizophrenia, it may be used in combination with medicaments claimed to be useful as treatments of schizophrenia including i) antipsychotics including typical antipsychotics (for example chlorpromazine, thioridazine, mesoridazine, fluphenazine, perphenazine, prochlorperazine, trifluoperazine, thiothixine, haloperidol, molindone and loxapine), atypical antipsychotics (for example clozapine, olanzapine, risperidone, quetiapine, aripirazole, ziprasidone, amisulpride and aripiprazole), glycine transporter 1 inhibitors and metabotropic receptor ligands; ii) drugs for extrapyramidal side effects, for example anticholinergics (such as benztropine, biperiden, procyclidine, and trihexyphenidyl) and dopaminergics (such as amantadine); iii) antidepressants including serotonin reuptake inhibitors (such as citalopram, escitalopram, fluoxetine, paroxetine, dapoxetine and sertraline), dual serotonin/noradrenaline reuptake inhibitors (such as venlafaxine, duloxetine and milnacipran), noradrenaline reuptake inhibitors (such as reboxetine), tricyclic antidepressants (such as amitriptyline, clomipramine, imipramine, maprotiline, nortriptyline and trimipramine), monoamine oxidase inhibitors (such as isocarboxazide, moclobemide, phenelzine and tranylcypromine), and others (such as buproprion, mianserin, mirtazepine, nefazodone and trazodone); iv) anxiolytics including benzodiazepines such as alprazolam and lorazepam; and v) cognitive enhancers for example cholinesterase inhibitors (such as tacrine, donepezil, rivastigmine and galantamine).

The invention thus provides, in a further aspect, a dosage form comprising a carrier tablet which carrier tablet is at least partially coated by a film comprising 1-{6-[(3-cyclobutyl-2,3,4,5-tetrahydro-1*H*-3-benzazepin-7-yl)oxy]-3-pyridinyl}-2-pyrrolidinone or a pharmaceutically acceptable salt thereof and a stabiliser, which dosage form further comprises an additional therapeutic agent or agents.

It will be apparent that the additional therapeutic agent may be present in the carrier tablet. Alternatively, as discussed above, a film containing additional therapeutic agents may be deposited on the carrier tablet. Where the carrier tablet has two recesses, one recess may contain the film containing 1-{6-[(3-cyclobutyl-2,3,4,5-tetrahydro-1*H*-3-benzazepin-7-yl)oxy]-3-pyidinyl}-2-pyrrolidinone or a pharmaceutically acceptable salt thereof and stabiliser, and the second recess may contain the film containing the additional therapeutic agent or agents. Alternatively, the films containing 1-{6-[(3-cyclobutyl-2,3,4,5-tetrahydro-1*H*-3-benzazepin-7-yl)oxy]-3-pyridinyl}-2-pyrrolidinone or a pharmaceutically acceptable salt thereof and stabiliser, and the additional therapeutic agent may be layered one on top of the other.

When 1-{6-[(3-cyclobutyl-2,3,4,5-tetrahydro-1*H*-3-benzazepin-7-yl)oxy]-3-pyridinyl}-2-pyrrolidinone or a pharmaceutically acceptable salt thereof is used in combination with a second therapeutic agent active against the same disease state the dose of 1-{6-[(3-cyclobutyl-2,3,4,5-tetrahydro-1*H*-3-benzazepin-7-yl)oxy]-3-pyridinyl}-2-pyrrolidinone (or its pharmaceutically acceptable salt) may differ from that when the 1-{6-[(3-cyclobutyl-2,3,4,5-tetrahydro-1*H*-3-benzazepin-7-yl)oxy]-3-pyridinyl}-2-pyrrolidinone (or its pharmaceutically acceptable salt) is formulated alone. Appropriate doses will be readily appreciated by those skilled in the art.

### Examples

### Example 1: Preparation of round tablets containing 0.05mg 1-{6-[(3-cyclobutyl-2,3,4,5-tetrahydro-1H-3-benzazepin-7-yl)oxy]-3-pyridinyl}-2-pyrrolidinone

The core components were passed through a nominal 30 mesh screen and then blended together in a suitable blender and compressed on a rotary tablet press to produce round biconcave tablets with a diameter of 9.525 mm and an approximately 0.8 mm deep trough on both sides of the tablet. Compression was followed by de-dusting and metal checking. The tablets were the transferred to a coating pan and coated to a target 4% (w/w) gain.

The composition of the carrier tablets is given below:

**Core**

| Component | Weight (mg) |
|---|---|
| Microcrystalline cellulose (Avicel PH-102) | 210 |
| Pregelatinized Starch (Starch 1500) | 21 |
| Magnesium Stearate | 2.3 |
| Total | 233.3 |

**Film Coat**

| Component | Weight (mg) |
|---|---|
| Opadry White YS-1-7003 | 9.3 |
| Purified Water | qs* |
| Total | 242.6 |

| | |
|---|---|
| *Removed during processing | |

Carrier solution was prepared by dissolving 4 g hydroxypropyl cellulose (Grade EF; HPC), 2 g anhydrous citric acid and 0.02 g butylated hydroxyanisole (BHA) in methanol, filtering through a 10 micron filter and then bringing the final volume to 100 ml with methanol.

1-{6-[(3-Cyclobutyl-2,3,4,5-tetrahydro-1*H*-3-benzazepin-7-yl)oxy]-3-pyridinyl}-2-pyrrolidinone was dissolved in the carrier solution with a sonicator until a uniform solution was obtained with a final concentration of 5 mg/g (w/w).

10 mg dosing solution was dispensed onto each tablet in an array of carrier tablets. The tablets were dried in a forced air oven at -40°C for 10-20 minutes.

The composition of the finished tablets is as follows:

| Component | Weight (mg) |
|---|---|
| 1-{6-[(3-Cyclobutyl-2,3,4,5-tetrahydro-1*H*-3-benzazepin-7-yl)oxy]-3-pyridinyl}-2-pyrrolidinone | 0.05 |
| Carrier (4% HPC/2% Citric acid/0.02% BHA in methanol*) | 0.6 |
| Carrier tablet | 242.6 |
| Total | 243.3 |

| | |
|---|---|
| * removed during manufacturing process | |

### Examples 2-4: Preparation of round tablets containing 0.002 mg, 0.01 mg or 0.2 mg 1-{6-[(3-cyclobutyl-2,3,4,5-tetrahydro-1H-3-benzazepin-7-yl)oxy]-3-pyridinyl}-2-pyrrolidinone

Tablets containing 0.002 mg, 0.01 mg or 0.2 mg 1-{6-[(3-cyclobutyl-2,3,4,5-tetrahydro-1*H*-3-benzazepin-7-yl)oxy]-3-pyridinyl}-2-pyrrolidinone were prepared in the manner described in Example 1 except that the concentration of 1-{6-[(3-cyclobutyl-2,3,4,5-tetrahydro-1*H*-3-benzazepin-7-yl)oxy]-3-pyridinyl}-2-pyrrolidinone in the dosing solution was varied.

### Example 5: Preparation of oval tablets containing 1 mg 1-{6-[(3-cyclobutyl-2,3,4,5-tetrahydro-1H-3-benzazepin-7-yl)oxy]-3-pyridinyl}-2-pyrrolidinone

The core components were passed through a nominal 30 mesh screen and then blended together in a suitable blender and compressed on a rotary tablet press to produce oval biconcave tablets with dimensions of 15.9 mm x 8 mm x 3.4 mm with an approximately 0.9 mm trough on both sides. Compression was followed by de-dusting and metal checking. The tablets were the transferred to a coating pan and coated to a target 4% (w/w) gain.

The composition of the carrier tablets is given below:

**Core**

| Component | Weight (mg) |
|---|---|
| Microcrystalline cellulose (Avicel PH-102) | 286.2 |
| Pregelatinized Starch (Starch 1500) | 28.6 |
| Magnesium Stearate | 3.2 |
| Total | 318.0 |

**Film Coat**

| Component | Weight (mg) |
|---|---|
| Opadry White 00F18484 | 12.7 |
| Purified Water | qs* |
| Total | 330.7 |

| | |
|---|---|
| *Removed during processing | |

Carrier solution was prepared by dissolving 4 g hydroxypropyl cellulose (Grade EF; HPC), 2 g anhydrous citric acid and 0.02 g butylated hydroxyanisole (BHA) in methanol, filtering through a 10 micron filter and then bringing the final volume to 100 ml with methanol.

1-{6-[(3-cyclobutyl-2,3,4,5-tetrahydro-1*H*-3-benzazepin-7-yl)oxy]-3-pyridinyl}-2-pyrrolidinone was dissolved in the carrier solution with a sonicator until a uniform solution was obtained with a final concentration of 35 mg/g (w/w).

28.5 mg dosing solution was dispensed onto each tablet in an array of carrier tablets. The tablets were dried in a forced air oven at ~50°C for 10-20 minutes. If necessary or desirable, the drying temperature may be raised e.g. to ~60°C and/or the duration of drying may be increased in order to ensure adequate evaporation of solvent.

The composition of the finished tablets is as follows:

| Component | Weight (mg) |
|---|---|
| 1-{6-[(3-Cyclobutyl-2,3,4,5-tetrahydro-1*H*-3-benzazepin-7-yl)oxy]-3-pyridinyl}-2-pyrrolidinone | 1 |
| Carrier (4% HPC/2% Citric acid/0.02% BHA in methanol*) | 1.72 |
| Carrier tablet | 330.7 |
| Total | 333.42 |

| | |
|---|---|
| * removed during manufacturing process | |

### Example 6: Preparation of round tablets containing 0.01mg 1-{6-[(3-cyclobutyl-2,3,4,5-tetrahydro-1H-3-benzazepin-7-yl)oxy]-3-pyridinyl}-2-pyrrolidinone

The core components were passed through a nominal 30 mesh screen and then blended together in a suitable blender and compressed on a rotary tablet press to produce round biconcave tablets with a diameter of 7.9 mm and an approximately 0.8 mm deep trough on both sides of the tablet. Compression was followed by de-dusting and metal checking. The tablets were the transferred to a coating pan and coated to a target 4% (w/w) gain.

The composition of the carrier tablets is given below:

**Core**

| Component | Weight (mg) |
|---|---|
| Microcrystalline cellulose (Avicel PH-102) | 162 |
| Pregelatinized Starch (Starch 1500) | 16.2 |
| Magnesium Stearate | 1.8 |
| Total | 180 |

**Film Coat**

| Component | Weight (mg) |
|---|---|
| Opadry White YS-1-7003 | 7.2 |
| Purified Water | qs* |
| Total | 187.2 |

| | |
|---|---|
| *Removed during processing | |

Carrier solution was prepared by dissolving 5 g hydroxypropyl cellulose (Grade EF; HPC), and 3 g anhydrous citric acid in methanol, filtering through a 10 micron filter and then bringing the final volume to 100 ml with methanol.

1-{6-[(3-Cyclobutyl-2,3,4,5-tetrahydro-1*H*-3-benzazepin-7-yl)oxy]-3-pyridinyl}-2-pyrrolidinone was dissolved in the carrier solution with a sonicator (and by also using a magnetic stirrer) until a uniform solution was obtained with a final concentration of 12.5 mg/g (w/w).

4 mg carrier solution was dispensed onto each tablet in an array of carrier tablets. The tablets were dried in a forced air oven at ~50°C for 10-20 minutes.

The composition of the finished tablets is as follows:

| Component | Weight (mg) |
|---|---|
| 1-{6-[(3-Cyclobutyl-2,3,4,5-tetrahydro-1*H*-3-benzazepin-7-yl)oxy]-3-pyridinyl}-2-pyrrolidinone | 0.01 |
| Carrier (5% HPC/3% Citric acid in methanol*) | 0.4 |
| Carrier tablet | 187.2 |
| Total | 187.6 |

| | |
|---|---|
| * removed during manufacturing process | |

### Examples 7-11: Preparation of round tablets containing 0.002 mg, 0.005 mg, 0.02mg, 0.05mg or 0.1 mg 1-{6-[(3-cyclobutyl-2,3,4,5-tetrahydro-1H-3-benzazepin-7-yl)oxy]-3-pyridinyl}-2-pyrrolidinone

Tablets containing 0.002 mg, 0.005 mg, 0.02 mg, 0.05mg or 0.1 mg 1-{6-[(3-cyclobutyl-2,3,4,5-tetrahydro-1*H*-3-benzazepin-7-yl)oxy]-3-pyridinyl}-2-pyrrolidinone were prepared in the manner described in Example 6 except that the concentration of 1-{6-[(3-cyctobutyl-2,3,4,5-tetrahydro-1*H*-3-benzazepin-7-yl)oxy]-3-pyridinyl}-2-pyrrolidinone in the carrier solution was varied.

The following examples (Example 12 and Example 13) are representative of an example tablet which may be prepared in accordance with the invention:

### Example 12: Preparation of a Orally Disintegrating Tablet (ODT) Carrier Substrate

### a) Preparation of ODT Carrier Substrate

StarLac and Neotame are passed through a nominal 20 mesh screen. The mixture and unsieved mint flavoring are transferred to a suitable blender and blended for approximately 10 minutes. Magnesium stearate is passed through a nominal 30 mesh screen, transferred to the blender and the entire mixture blended for approximately 2 minutes. The weights of material used are calculated from the percentage weights given in Table A. The blend is compressed to meet the desired specifications (for example round, biconcave tablets, range in diameter from ~8 mm to ~9.5 mm) on a suitable rotary press utilizing appropriate tablet tooling. The tablets are passed through a de-duster and metal checker.

**Table A**

| COMPOSITION | SPECIFICATION | % (w/w) | FUNCTION |
|---|---|---|---|
| StarLac* | Non Compendial | 98.5% | Diluent |
| Mint Flavoring | Non Compendial | 0.9% | Flavoring |
| Neotame | NF | 0.1% | Sweetener |
| Magnesium Stearate | Ph. Eur/USP-NF/JP | 0.5% | Lubricant |

| | | | |
|---|---|---|---|
| * StarLac: mixture of 85% alpha-lactose monohydrate (Ph. Eur./USP-NF) and 15% maize starch (Ph. Eur./USP-NF) | | | |

### b) Preparation of ethylcellulose coat for application to tablet by Pad-Printing

Ethylcellulose is dissolved in methanol with stirring, and triethyl citrate added. The weights of material used are calculated from the percentage weights given in Table B. Sufficient methanol is added to bring to target on w/w basis. The solution is transferred to the ink cup of a pad printing machine equipped with a suitable image cliché with a round image, slightly smaller diameter then the actual tablet diameter. A suitable polymer pad is installed to match the cliché image plate. Tablets are presented to the pad printer in a defined array, matching the cliché. The pad printer may apply 2-4 tamps to the carrier tablet to apply a coat that will provide a protective layer to mitigate solvent infiltration into the uncoated carrier substrate during the liquid dispensing process.

**Table B**

| COMPOSITION | SPECIFICATION | % (w/w) | FUNCTION |
|---|---|---|---|
| Ethylcellulose | NF | 30 | Protective barrier coat |
| Triethyl Citrate | Ph. Eur./USP-NF | 1.67 | Plasticiser |
| Methanol | Ph. Eur./USP-NF | qs*** to 100 | vehicle |

| | | | |
|---|---|---|---|
| *** Methanol eliminated by evaporation. | | | |

### Example 13: Alternative preparation of an Orally Disintegrating Tablet (ODT) carrier substrate

Mannitol, crospovidone XL, xylitol and Neotame are passed through a nominal 20 mesh screen, the mixture and the unsieved Mint Flavoring transferred to a suitable blender and blended for approximately 10 minutes. Magnesium stearate and colloidal silicon dioxide are passed through a nominal 30 mesh screen, transferred to the blender and the entire mixture blended for approximately 2 minutes. The weights of material used are calculated from the percentage weights given in Table C. The blend is compressed to meet the desired specifications (for example round, biconcave tablets, range in diameter from -8 mm to -9.5 mm) on a suitable rotary press utilizing an appropriate tablet tooling. The tablets are passed through a de-duster and metal checker.

An ethylcellulose coat may be prepared and applied as described for Example 12.

**Table C**

| COMPOSITION | SPECIFICATION | % (w/w) | FUNCTION |
|---|---|---|---|
| Mannitol (Grade 300 Direct Compression) | Ph. Eur/USP-NF/JP | 73.15% | Diluent/sweetener |
| Crospovidone XL | Ph. Eur/USP-NF | 20.00% | Disintegrant |
| Xylitol (Grade 300 for Direct Compression) | Ph. Eur/USP-NF/JP | 5.00% | Diluent/sweetener |
| Mint Flavoring | Non Compendial | 0.90% | Flavoring |
| Neotame | NF | 0.10% | Sweetener |
| Magnesium Stearate | Ph. Eur/USP-NF/JP | 0.75% | Lubricant |
| Colloidal silicon dioxide | Ph. Eur/USP-NF/JP | 0.10% | Lubricant |

**PROPERTIES:** The stability of the drug substance in the tablets may be tested as set out below:
Dissolve 5 tablets in diluent (1:9 acetonitrile: 50 mM potassium dihydrogen orthophosphate, adjusted to pH 3 with orthophosphoric acid) to produce a final concentration of active agent of between 1-10 µg/ml and sonicate for 10 minutes. Check for complete disintegration and sonicate further if required. Allow to cool to ambient temperature and then centrifuge an aliquot of the sample at 14,000 rpm. Prepare samples using placebo tablets to act as control samples.

Using the following instrument conditions, equilibrate the chromatographic system with 95% A, 5%B. Record the chromatograms for the sample and placebo preparations.
Column: XBridge C18 3 µM 15 cm x 4.6 mm i.d.
Column Temperature: 40°C
Mobile Phase A: 10 mM ammonium bicarbonate, pH 10 with ammonia
Mobile Phase B: Acetonitrile
Flow Rate: 1 ml/min
Detector wavelength: 250 nm
Injection volume: 100 µl
Gradient Profile:

| Time (min) | %A | %B |
|---|---|---|
| 0 | 95 | 5 |
| 5 | 95 | 5 |
| 30 | 30 | 70 |
| 30.1 | 95 | 5 |

Following comparison of the chromatograms to identify impurities/degradation products, the percentage content of each impurity/degradation product in the control and sample injections can be calculated by dividing the area of the impurity/degradation product peak by the summed total of the peak for 1-{6-[(3-cyclobutyl-2,3,4,5-tetrahydro-1*H*-3-benzazepin-7-yl)oxy]-3-pyridinyl}-2-pyrrolidinone and all impurities/degradation products, and multiplying by 100.

The total impurity/degradation product content can be calculated by summing the percentage content of each impurity/degradation product present. Typically, only impurities/degradation products present in an amount of greater than or equal to 0.05 or 0.03% are included in the calculation of total impurity/degradation product content.

## Claims

1. A dosage form for oral administration which comprises a carrier tablet, which carrier tablet is at least partially covered by a film comprising:
a) 1-{6-[(3-cyclobutyl-2,3,4,5-tetrahydro-1*H*-3-benzazepin-7-yl)oxy]-3-pyridinyl}-2-pyrrolidinone or a pharmaceutically acceptable salt thereof, and
b) a stabiliser that reduces degradation of 1-{6-[(3-cyclobutyl-2,3,4,5-tetrahydro-1*H*-3-benzazepin-7-yl)oxy]-3-pyridinyl}-2-pyrrolidinone in the dosage form, when compared to a dosage form lacking said stabiliser;
wherein said stabiliser is selected from the group consisting of citric acid, malic acid, ascorbic acid and its salts, sodium bicarbonate, butylated hydroxyanisole and butylated hydroxytoluene.

2. A dosage form for oral administration according to claim 1, wherein the film additionally contains a film former.

3. A dosage form for oral administration according to claim 2, wherein the film former is hydroxypropylcellulose.

4. A dosage form for oral administration according to claim 1, 2 or 3, wherein said carrier tablet has at least one recess.

5. A dosage form for oral administration according to claim 4, wherein said film is present in a recess on said carrier tablet.

6. A dosage form for oral administration according to any one of claims 1 to 5, wherein there is substantially no absorption of the 1-{6-[(3-cyclobutyl-2,3,4,5-tetrahydro-1*H*-3-benzazepin-7-yl)oxy]-3-pyridinyl}-2-pyrrolidinone or the pharmaceutically acceptable salt thereof by the carrier tablet.

7. A dosage form for oral administration according to claim 6, wherein said carrier tablet is coated.

8. A dosage form for oral administration according to any one of the preceding claims, which contains between 1 µg and 1 mg 1-{6-[(3-cyclobutyl-2,3,4,5-tetrahydro-1*H*-3-benzazepin-7-yl)oxy]-3-pyridinyl}-2-pyrrolidinone, when measured as the amount of free base present.

9. A dosage form for oral administration according to any one of the preceding claims, which comprises 1-{6-[(3-cyclobutyl-2,3,4,5-tetrahydro-1*H*-3-benzazepin-7-yl)oxy]-3-pyridinyl}-2-pyrrolidinone as a free base.

10. A dosage form for oral administration according to any one of the preceding claims, wherein said stabiliser is citric acid and optionally butylated hydroxyanisole.

11. A dosage form for oral administration according to any one of claims 1 to 7, wherein said stabiliser is citric acid, and wherein the molar ratio of the free base 1-{6-[(3-cyclobutyl-2,3,4,5-tetrahydro-1*H*-3-benzazepin-7-yl)oxy]-3-pyridinyl}-2-pyrrolidinone to citric acid is in the range 1.5 : 1 to 1 : 500.

12. A dosage form for oral administration according to any one of the preceding claims, wherein the dosage form and/or the film contains between 2 µg and 100 µg 1-{6-[(3-cyclobutyl-2,3,4,5-tetrahydro-1*H*-3-benzazepin-7-yl)oxy]-3-pyridinyl}-2-pyrrolidinone, when measured as the amount of free base present.

13. A dosage form for oral administration according to any one of the preceding claims, wherein said dosage form is further coated.

14. A dosage form for oral administration according to any one of the preceding claims wherein the carrier tablet is a tablet comprising microcrystalline cellulose (e.g. Avicel PH-102), pregelatinised starch (e.g. Starch 1500) and magnesium stearate.

15. A dosage form for oral administration according to claim 14 wherein the carrier tablet comprises 90% microcrystalline cellulose (e.g. Avicel PH-102), 9% pregelatinised starch (e.g. Starch 1500) and 1% magnesium stearate.

16. A method of producing a dosage form for oral administration as defined in claim 1, comprising dispensing a solution or suspension of 1-{6-[(3-cyclobutyl-2,3,4,5-tetrahydro-1*H-*3-benzazepin-7-yl)oxy]-3-pyridinyl}-2-pyrrolidinone or a pharmaceutically acceptable salt thereof and a stabiliser onto a carrier tablet.

17. A method according to claim 16, wherein the solution or suspension of the 1-{6-[(3-cyclobutyl-2,3,4,5-tetrahydro-1*H*-3-benzazepin-7-yl)oxy]-3-pyridinyl}-2-pyrrolidinone or the pharmaceutically acceptable salt thereof and the stabiliser is prepared using an organic solvent being methanol, ethanol, acetone, acetic acid or methylene chloride.

18. A method according to claim 16 or 17, wherein the stabiliser is citric acid, and it is present in the solution or suspension in an amount between 2-3% w/v.

19. A method according to claim 16, 17 or 18, wherein, in the dosage form, the carrier tablet is at least partially covered by a film as defined in claim 1, and wherein the film additionally contains a film former which is hydroxypropylcellulose, and wherein, in the method, the hydroxypropylcellulose is present in the solution or suspension in an amount between 4-6% w/v.

20. A method according to claim 16, 17, 18 or 19, wherein the carrier tablet and the dispensed solution/suspension is heated to evaporate excessive liquid and to result in the formation of a film upon at least a part of the surface of the carrier tablet.

21. A method according to any one of claims 16 to 20, wherein the carrier tablet used in the method has a recess or depression that provides a basin for the solution or suspension of the 1-{6-[(3-cyclobutyl-2,3,4,5-tetrahydro-1*H*-3-benzazepin-7-yl)oxy]-3-pyridinyl}-2-pyrrolidinone or the pharmaceutically acceptable salt thereof and the stabiliser to land after being dispensed.

22. A method according to claim 21 in which biconcave tablets having recesses on two faces of the tablet are employed.

23. Use of 1-{6-[(3-cyclobutyl-2,3,4,5-tetrahydro-1*H*-3-benzazepin-7-yl)oxy]-3-pyridinyl}-2-pyrrolidinone or a pharmaceutically acceptable salt thereof in the manufacture of a dosage form for oral administration as defined in any one of claims 1 to 15 for the treatment of neurological diseases.

24. Use of 1-{6-[(3-cyclobutyl-2,3,4,5-tetrahydro-1*H*-3-benzazepin-7-yl)oxy]-3-pyridinyl}-2-pyrrolidinone or a pharmaceutically acceptable salt thereof according to claim 23 wherein the neurological disease is multiple sclerosis.

25. A dosage form for oral administration as defined in any one of claims 1 to 15 for use in the treatment of neurological diseases.

26. A dosage form according to claim 25 wherein the neurological disease is multiple sclerosis.

## Patentansprüche

1. Eine Dosierungsform zur oralen Verabreichung, die eine Trägertablette umfasst, wobei die Trägertablette mindestens teilweise durch einen Film, umfassend:
a) 1-{6-[(3-Cyclobutyl-2,3,4,5-tetrahydro-1*H*-3-benzazepin-7-yl)oxy]-3-pyridinyl}-2-pyrrolidinon oder ein pharmazeutisch verträgliches Salz davon, und
b) einen Stabilisator, der den Abbau von 1-{6-[(3-Cyclobutyl-2,3,4,5-tetrahydro-1*H*-3-benzazepin-7-yl)oxy]-3-pyridinyl}-2-pyrrolidinon in der Dosierungsform verringert, im Vergleich mit einer Dosierungsform, die den Stabilisator nicht enthält,
bedeckt ist;
wobei der Stabilisator ausgewählt ist aus der Gruppe bestehend aus Citronensäure, Äpfelsäure, Ascorbinsäure und Salzen davon, Natriumbicarbonat, butyliertem Hydroxyanisol und butyliertem Hydroxytoluol.

2. Eine Dosierungsform zur oralen Verabreichung gemäß Anspruch 1, wobei der Film zusätzlich einen Filmbildner enthält.

3. Eine Dosierungsform zur oralen Verabreichung gemäß Anspruch 2, wobei der Filmbildner Hydroxypropylcellulose ist.

4. Eine Dosierungsform zur oralen Verabreichung gemäß Anspruch 1, 2 oder 3, wobei die Trägertablette mindestens eine Aussparung aufweist.

5. Eine Dosierungsform zur oralen Verabreichung gemäß Anspruch 4, wobei sich der Film in einer Aussparung auf der Trägertablette befindet.

6. Eine Dosierungsform zur oralen Verabreichung gemäß einem der Ansprüche 1 bis 5, wobei im Wesentlichen keine Absorption des 1-{6-[(3-Cyclobutyl-2,3,4,5-tetrahydro-1*H*-3-benzazepin-7-yl)oxy]-3-pyridinyl}-2-pyrrolidinons oder des pharmazeutisch verträglichen Salzes davon durch die Trägertablette stattfindet.

7. Eine Dosierungsform zur oralen Verabreichung gemäß Anspruch 6, wobei die Trägertablette beschichtet ist.

8. Eine Dosierungsform zur oralen Verabreichung gemäß einem der vorangehenden Ansprüche, die zwischen 1 µg und 1 mg 1-{6-[(3-Cyclobutyl-2,3,4,5-tetrahydro-1*H*-3-benzazepin-7-yl)oxy]-3-pyridinyl}-2-pyrrolidinon enthält, gemessen als die Menge an vorhandener freier Base.

9. Eine Dosierungsform zur oralen Verabreichung gemäß einem der vorangehenden Ansprüche, die 1-{6-[(3-Cyclobutyl-2,3,4,5-tetrahydro-1*H*-3-benzazepin-7-yl)oxy]-3-pyridinyl}-2-pyrrolidinon als eine freie Base umfasst.

10. Eine Dosierungsform zur oralen Verabreichung gemäß einem der vorangehenden Ansprüche, wobei der Stabilisator Citronensäure und gegebenenfalls butyliertes Hydroxyanisol ist.

11. Eine Dosierungsform zur oralen Verabreichung gemäß einem der Ansprüche 1 bis 7, wobei der Stabilisator Citronensäure ist, und wobei das Molverhältnis der freien Base 1-{6-[(3-Cyclobutyl-2,3,4,5-tetrahydro-1*H*-3-benzazepin-7-yl)oxy]-3-pyridinyl}-2-pyrrolidinon zu Citronensäure im Bereich von 1,5:1 bis 1:500 liegt.

12. Eine Dosierungsform zur oralen Verabreichung gemäß einem der vorangehenden Ansprüche, wobei die Dosierungsform und/oder der Film zwischen 2 µg und 100 µg 1-{6-[(3-Cyclobutyl-2,3,4,5-tetrahydro-1*H*-3-benzazepin-7-yl)oxy]-3-pyridinyl}-2-pyrrolidinon enthält, gemessen als die Menge an vorhandener freier Base.

13. Eine Dosierungsform zur oralen Verabreichung gemäß einem der vorangehenden Ansprüche, wobei die Dosierungsform außerdem beschichtet ist.

14. Eine Dosierungsform zur oralen Verabreichung nach einem der vorangehenden Ansprüche, wobei die Trägertablette eine Tablette ist, die mikrokristalline Cellulose (z.B. Avicel PH-102), Quellstärke (z.B. Stärke 1500) und Magnesiumstearat umfasst.

15. Eine Dosierungsform zur oralen Verabreichung nach Anspruch 14, wobei die Trägertablette 90% mikrokristalline Cellulose (z.B. Avicel PH-102), 9% Quellstärke (z.B. Stärke 1500) und 1% Magnesiumstearat umfasst.

16. Ein Verfahren zur Herstellung einer Dosierungsform zur oralen Verabreichung, wie in Anspruch 1 definiert, umfassend das Abgeben einer Lösung oder Suspension des 1-{6-[(3-Cyclobutyl-2,3,4,5-tetrahydro-1*H*-3-benzazepin-7-yl)oxy]-3-pyridinyl}-2-pyrrolidinons oder eines pharmazeutisch verträglichen Salzes davon und eines Stabilisators auf eine Trägertablette.

17. Ein Verfahren gemäß Anspruch 16, wobei die Lösung oder Suspension des 1-{6-[(3-Cyclobutyl-2,3,4,5-tetrahydro-1*H*-3-benzazepin-7-yl)oxy]-3-pyridinyl}-2-pyrrolidinons oder des pharmazeutisch verträglichen Salzes davon und des Stabilisators unter Verwendung eines organischen Lösungsmittels, welches Methanol, Ethanol, Aceton, Essigsäure oder Methylenchlorid ist, hergestellt wird.

18. Ein Verfahren gemäß Anspruch 16 oder 17, wobei der Stabilisator Citronensäure ist, und sie in der Lösung oder Suspension in einer Menge zwischen 2-3% Gew./Vol. vorhanden ist.

19. Ein Verfahren gemäß Anspruch 16, 17 oder 18, wobei, in der Dosierungsform, die Trägertablette zumindest teilweise mit einem wie in Anspruch 1 definierten Film bedeckt ist und wobei der Film zusätzlich einen Filmbildner, der Hydroxypropylcellulose ist, enthält und wobei in dem Verfahren die Hydroxypropylcellulose in der Lösung oder Suspension in einer Menge zwischen 4-6% Gew./Vol. vorhanden ist.

20. Ein Verfahren gemäß Anspruch 16, 17, 18 oder 19, wobei die Trägertablette und die abgegebene Lösung/Suspension erwärmt wird, um überschüssige Flüssigkeit zu verdampfen und um zur Bildung eines Films auf mindestens einem Teil der Oberfläche der Trägertablette zu führen.

21. Ein Verfahren gemäß einem der Ansprüche 16 bis 20, wobei die Trägertablette, die in dem Verfahren verwendet wird, eine Aussparung oder Vertiefung hat, die eine Mulde für die Lösung oder Suspension des 1-{6-[(3-Cyclobutyl-2,3,4,5-tetrahydro-1*H*-3-benzazepin-7-yl)oxy]-3-pyridinyl}-2-pyrrolidinons oder des pharmazeutisch verträglichen Salzes davon und des Stabilisators bereitstellt, in dem diese landen kann, nachdem sie abgegeben wurde.

22. Ein Verfahren gemäß Anspruch 21, bei dem bikonkave Tabletten mit Aussparungen auf zwei Flächen der Tablette verwendet werden.

23. Verwendung von 1-{6-[(3-Cyclobutyl-2,3,4,5-tetrahydro-1*H*-3-benzazepin-7-yl)oxy]-3-pyridinyl}-2-pyrrolidinon oder eines pharmazeutisch verträglichen Salzes davon bei der Herstellung einer Dosierungsform zur oralen Verabreichung, wie in einem der Ansprüche 1 bis 15 definiert, zur Behandlung von neurologischen Erkrankungen.

24. Verwendung von 1-{6-[(3-Cyclobutyl-2,3,4,5-tetrahydro-1*H*-3-benzazepin-7-yl)oxy]-3-pyridinyl}-2-pyrrolidinon oder eines pharmazeutisch verträglichen Salzes davon nach Anspruch 23, wobei die neurologische Erkrankung Multiple Sklerose ist.

25. Eine Dosierungsform zur oralen Verabreichung, wie in einem der Ansprüche 1 bis 15 definiert, zur Verwendung bei der Behandlung von neurologischen Erkrankungen.

26. Eine Dosierungsform gemäß Anspruch 25, wobei die neurologische Erkrankung Multiple Sklerose ist.

## Revendications

1. Forme posologique pour administration orale qui comprend un comprimé de support, comprimé de support qui est couvert au moins partiellement d'un film comprenant :
a) de la 1-{6-[(3-cyclobutyl-2,3,4,5-tétrahydro-1*H-*3-benzazépine-7-yl)oxy]-3-pyridinyl}-2-pyrrolidone ou un de ses sels pharmaceutiquement acceptables, et
b) un stabilisant qui réduit la dégradation de la 1-{6-[(3-cyclobutyl-2,3,4,5-tétrahydro-1*H*-3-benzazépine-7-yl)oxy]-3-pyridinyl}-2-pyrrolidone dans la forme posologique, comparativement à une forme posologique dépourvue dudit stabilisant ;
dans laquelle ledit stabilisant est choisi dans le groupe consistant en l'acide citrique, l'acide malique, l'acide ascorbique et ses sels, le bicarbonate de sodium, le butylhydroxyanisole et le butylhydroxytoluène.

2. Forme posologique pour administration orale suivant la revendication 1, dans laquelle le film contient en outre un agent filmogène.

3. Forme posologique pour administration orale suivant la revendication 2, dans laquelle l'agent filmogène est l'hydroxypropylcellulose.

4. Forme posologique pour administration orale suivant la revendication 1, 2 ou 3, dans laquelle ledit comprimé de support comporte au moins un évidement.

5. Forme posologique pour administration orale suivant la revendication 4, dans laquelle ledit film est présent dans un évidement sur ledit comprimé de support.

6. Forme posologique pour administration orale suivant l'une quelconque des revendications 1 à 5, dans laquelle il n'existe pratiquement aucune absorption de la 1-{6-[(3-cyclobutyl-2,3,4,5-tétrahydro-1*H*-3-benzazépine-7-yl)oxy]-3-pyridinyl]-2-pyrrolidone ou de son sel pharmaceutiquement acceptable par le comprimé de support.

7. Forme posologique pour administration orale suivant la revendication 6, dans laquelle ledit comprimé de support est enrobé.

8. Forme posologique pour administration orale suivant l'une quelconque des revendications précédentes, qui contient de 1 µg à 1 mg de 1-{6-[(3-cyclobutyl-2,3,4,5-tétrahydro-1*H*-3-benzazépine-7-yl)oxy]-3-pyridinyl}-2-pyrrolidone, lors de la mesure sous forme de la quantité de base libre présente.

9. Forme posologique pour administration orale suivant l'une quelconque des revendications précédentes, qui comprend de la 1-{6-[(3-cyclobutyl-2,3,4,5-tétrahydro-1*H*-3-benzazépine-7-yl)oxy]-3-pyridinyl}-2-pyrrolidone sous forme de base libre.

10. Forme posologique pour administration orale suivant l'une quelconque des revendications précédentes, dans laquelle ledit stabilisant est l'acide citrique et le butylhydroxy-anisole.

11. Forme posologique pour administration orale suivant l'une quelconque des revendications 1 à 7, dans laquelle ledit stabilisant est l'acide citrique, et dans laquelle le rapport molaire de la base libre de 1-{6-[(3-cyclobutyl-2,3,4,5-tétrahydro-1*H*-3-benzazépine-7-yl)oxy]-3-pyridinyl}-2-pyrrolidone à l'acide citrique est compris dans l'intervalle de 1,5:1 à 1:500.

12. Forme posologique pour administration orale suivant l'une quelconque des revendications précédentes, ladite forme posologique et/ou le film contenant de 2 µg à 100 µg de 1-{6-[(3-cyclobutyl-2,3,4,5-tétrahydro-1*H*-3-benzazépine-7-yl)oxy]-3-pyridinyl}-2-pyrrolidone, lors de la mesure sous forme de la quantité de base libre présente.

13. Forme posologique pour administration orale suivant l'une quelconque des revendications précédentes, ladite forme posologique étant en outre enrobée.

14. Forme posologique pour administration orale suivant l'une quelconque des revendications précédentes, dans laquelle le comprimé de support est un comprimé comprenant de la cellulose microcristalline (par ex. Avicel PH-102), de l'amidon prégélatinisé (par ex. Starch 1500) et du stéarate de magnésium.

15. Forme posologique pour administration orale suivant la revendication 14, dans laquelle le comprimé de support comprend 90 % de cellulose microcristalline (par ex. Avicel PH-102), 9 % d'amidon prégélatinisé (par ex. Starch 1500) et 1 % de stéarate de magnésium.

16. Procédé pur produire une forme posologique pour administration orale telle que définie dans la revendication 1, comprenant la distribution d'une solution ou suspension de 1-{6-[(3-cyclobutyl-2,3,4,5-tétrahydro-1*H*-3-benzazépine-7-yl)oxy]-3-pyridinyl}-2-pyrrolidone ou d'un de ses sels pharmaceutiquement acceptables et d'un stabilisant sur un comprimé de support.

17. Procédé suivant la revendication 16, dans lequel la solution ou suspension de la 1-{6-[(3-cyclobutyl-2,3,4,5-tétrahydro-1*H*-3-benzazépine-7-yl)oxy]-3-pyridinyl}-2-pyrrolidone ou de son sel pharmaceutiquement acceptable et du stabilisant est préparée en utilisant un solvant organique consistant en méthanol, éthanol, acétone, acide acétique ou chlorure de méthylène.

18. Procédé suivant la revendication 16 ou 17, dans lequel le stabilisant est l'acide citrique et est présent dans la solution ou suspension en une quantité comprise entre 2 et 3 % en poids/volume.

19. Procédé suivant la revendication 16, 17 ou 18, dans lequel, dans la forme posologique, le comprimé de support est couvert au moins partiellement d'un film tel que défini dans la revendication 1, et dans lequel le film contient en outre un agent filmogène qui est l'hydroxypropylcellulose, et dans lequel, dans le procédé, l'hydroxypropylcellulose est présente dans la solution ou suspension en une quantité comprise entre 4 et 6 % en poids/volume.

20. Procédé suivant la revendication 16, 17, 18 ou 19, dans lequel le comprimé de support et la solution/suspension distribuée sont chauffés pour faire s'évaporer l'excès de liquide et pour qu'il en résulte la formation d'un film sur au moins une partie de la surface du comprimé de support.

21. Procédé suivant l'une quelconque des revendications 16 à 20, dans lequel le comprimé de support utilisé dans la procédé comprend un évidement ou une dépression qui fournit une poche pour la solution ou suspension de la 1-{6-[(3-cyclobutyl-2,3,4,5-tétrahydro-1*H*-3-benzazépine-7-yl)oxy]-3-pyridinyl}-2-pyrrolidone ou de son sel pharmaceutiquement acceptable et le stabilisant à déposer après distribution.

22. Procédé suivant la revendication 21, dans lequel des comprimés biconcaves comportant des évidements sur deux faces du comprimé sont utilisés.

23. Utilisation de 1-{6-[(3-cyclobutyl-2,3,4,5-tétrahydro-1*H*-3-benzazépine-7-yl)oxy]-3-pyridinyl}-2-pyrrolidone ou d'un de ses sels pharmaceutiquement acceptables dans la production d'une forme posologique pour administration orale telle que définie dans l'une quelconque des revendications 1 à 15, pour le traitement de maladies neurologiques.

24. Utilisation de 1-{6-[(3-cyclobutyl-2,3,4,5-tétrahydro-1*H*-3-benzazépine-7-yl)oxy]-3-pyridinyl}-2-pyrrolidone ou d'un de ses sels pharmaceutiquement acceptables suivant la revendication 23, dans laquelle la maladie neurologique est la sclérose en plaques.

25. Forme posologique pour administration orale telle que définie dans l'une quelconque des revendications 1 à 15 pour une utilisation dans le traitement de maladies neurologiques.

26. Forme posologique suivant la revendication 25, dans laquelle la maladie neurologique est la sclérose en plaques.
